# EUROPEAN PATENT SPECIFICATION

(11) **EP 2 797 654 B1**
(45) Date of publication and mention of the grant of the patent: **19.07.2017**
(21) Application number: 12813492.1
(22) Date of filing: 26.11.2012
(51) Int. Cl.: A61M 16/08

(54) **ENDOTRACHEAL TUBE ELBOW CONNECTOR**
ENDOTRACHEALTUBUS-KNIEVERBINDUNG
RACCORD COUDÉ DE TUBE ENDOTRACHÉAL

(30) Priority: 27.12.2011 US 201161580351 P
(43) Date of publication of application: 05.11.2014
(73) Proprietor: Koninklijke Philips N.V., 5656 AE Eindhoven (NL)
(72) Inventor: STEGMAN, Steven, Charles, NL-5656 AE Eindhoven (NL); DUICH, Michael, Burton, NL-5656 AE Eindhoven (NL)
(74) Representative: Steffen, Thomas
(86) International application number: PCT/IB2012/056750
(87) International publication number: WO 2013/098674

(56) References cited:
- WO-A1-2006/131719
- GB-A- 2 069 849
- GB-A- 2 367 009
- US-A- 4 054 135
- US-A1- 2006 266 365
- US-A1- 2010 163 051
- US-B1- 6 494 203

## Description

### Technical Field

The inventive concepts described herein generally relate to a connector for a respiratory mask. More particularly, the inventive concepts relate to a connector for a respiratory mask, the connector accommodating an endotracheal tube and removable from around the endotracheal tube.

### Background

Positive pressure ventilation is a medical therapy that is used to support or replace the normal breathing cycle of patients. Mechanical ventilators provide appropriate pressure and volume of gases and/or gas mixtures. The interface between the patient and the breathing circuit including the mechanical ventilator can be defined as invasive or non-invasive. Non-invasive ventilation (NIV) utilizes a respiratory mask which encloses the nose and/or the mouth. An air tight or nearly air tight seal is provided between the respiratory mask and the patient by way of compliance material, straps and/or plastic framework. Invasive ventilation (IV) utilizes an artificial airway which extends into the upper lungs of the patient either through the mouth (endotracheal tube) or via a surgical opening in the front of the windpipe (tracheoscopy tube). Typically, when an endotracheal tube is inserted into a patient, a laryngoscope is utilized to physically open the airway of the patient.

Typically, non-invasive ventilation may be first administered to a patient in respiratory distress. As the respiratory stress continues or worsens, or in the case of preparation for major surgery, invasive ventilation including intubation may thereafter be necessary. In most cases, it is desirable not to interrupt NIV therapy during intubation. Also, removal of the respiratory mask from the patient may be desired during the transition from non-invasive ventilation to invasive ventilation.

Current adapters and like devices concerning invasive therapy include, e.g., G.B. Patent Application No. 2069849 to Searle & Co., U.S. Patent Application No. 2010/163051 to Brewer et. al., U.S. Patent No. 6494203 to Palmer, GB Patent Application No. 2367009 to Craft et. al., and U.S Patent No. 4054135 to Berman. GB2069849 discloses a swivel adaptor comprising a patient port to accept a face mask or an endotracheal tube, a machine port, and a third port adapted for receiving a fresh gas inlet tube by a snap fit or a suction catheter by insertion.

Thus, there is a need in the art for a respiratory mask interface that enables transition from non-invasive ventilation to invasive ventilation, that simplifies removal of the respiratory mask during the transition, and that simplifies insertion of the endotracheal tube without substantial interruption of ventilation.

### Summary

In accordance with an example embodiment, a connector for a respiratory mask is provided, the connector including a first end portion defining a first opening to connect with the respiratory mask; a second end portion defining a second opening to receive a non-invasive (NIV) tube; and an elbow portion between the first and second end portions, the elbow portion defining a port for receiving a tube, wherein at least one of the first end portion, the second end portion and the elbow portion is openable for removal of the connector from around the tube inserted in the port.

In accordance with another example embodiment, a connector for a respiratory mask is provided, the connector including a first end portion defining a first opening to connect with the respiratory mask; a second end portion defining a second opening to receive a non-invasive (NIV) tube; and a junction portion disposed between the first and second end portions, the junction portion defining a port for receiving a tube, wherein at least a portion of the connector is openable for removal of the connector from around the tube inserted in the port.

It should be appreciated that all combinations of the foregoing concepts and additional concepts discussed in greater detail below (provided such concepts are not mutually inconsistent) are contemplated as being part of the inventive concepts disclosed herein. In particular, all combinations of claimed subject matter appearing at the end of this disclosure are contemplated as being part of the inventive concepts disclosed herein. It should also be appreciated that terminology explicitly employed herein that also may appear in any disclosure incorporated by reference should be accorded a meaning most consistent with the particular concepts disclosed herein.

### Brief Description of the Drawings

In the drawings, like reference characters generally refer to the same parts throughout the different views. Also, the drawings are not necessarily to scale, emphasis instead generally being placed upon illustrating the principles of the inventive concepts.
FIG. 1 is a diagram illustrating a respiratory mask having a connector as an interface between an NIV tube and the respiratory mask, the connector having a port with an endotracheal tube inserted therein, according to a representative embodiment.
FIG. 2 is a diagram illustrating a connector with a hingeable end portion, according to a representative embodiment.
FIG. 3 is a diagram illustrating a connector with a hingeable end portion having mating surfaces respectively including ridges and recesses, according to a representative embodiment.
FIG. 4 is a diagram illustrating a connector with a hingeable end portion having mating surfaces respectively including ridges and recesses made of flexible sealing material, according to a representative embodiment.
FIG. 5 is a diagram illustrating a connector that is hingeable and having first and second end portions that are openable, according to a representative embodiment.
FIG. 6 is a diagram illustrating a connector having a channel and a port, including a removable seal in the channel and port, according to a representative embodiment.
FIG. 7 is a diagram illustrating a connector having a channel and a port, including a removable seal in the channel and port, the removable seal in the port having a slit, according to a representative embodiment.

### Detailed Description

In the following detailed description, for purposes of explanation and not limitation, representative embodiments disclosing specific details are set forth in order to provide a thorough understanding of the present teachings. However, it will be apparent to one having ordinary skill in the art having had the benefit of the present disclosure that other embodiments according to the present teachings that depart from the specific details disclosed herein remain within the scope of the appended claims. Moreover, descriptions of well-known apparatuses and methods may be omitted so as to not obscure the description of the representative embodiments. Such methods and apparatuses are clearly within the scope of the present teachings.

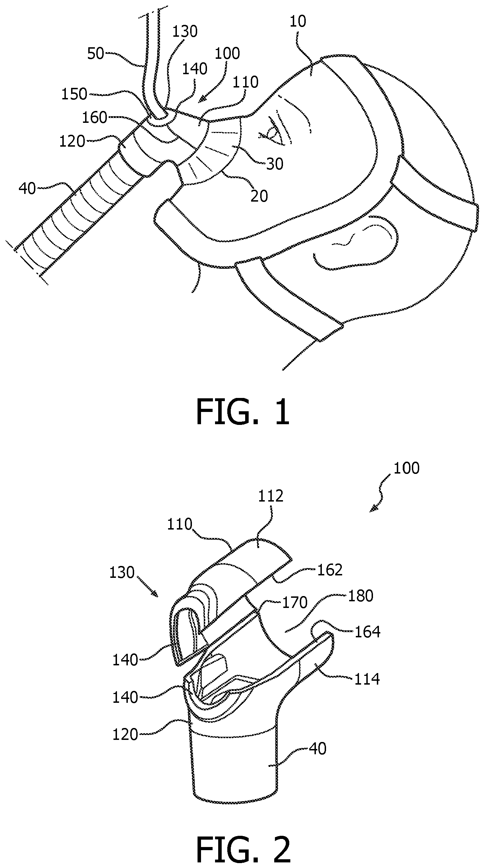

FIG. 1 is a diagram illustrating a respiratory mask having a connector as an interface between an NIV tube and the respiratory mask, the connector having a port with an endotracheal tube inserted therein, according to a representative embodiment.

Referring to FIG. 1, respiratory mask 10 is secured to the patient via straps and encloses the nose and/or mouth of the patient. An air tight or nearly air tight seal may be provided between respiratory mask 10 and the patient by way of compliance material, and/or plastic framework. The front of respiratory mask 10 includes an opening 20, and a hub 30 that is integral with respiratory mask 10. Connector 100 includes a first end portion 110 that may be of general tubular cross-section and that has a first opening (not shown). The first end portion 110 of connector 100 is removably insertable into hub 30. Hub 30 provides an air tight interface or passageway for gas flow between first end portion 110 of connector 100 and respiratory mask 10. The combination of respiratory mask 10 and hub 30 may be a Philips NIV mask with removable hub system.

Connector 100 further includes a second end portion 120 that may be of general tubular cross-section and that has a second opening (not shown). Second end portion 120 is removably connectable with a first end of non-invasive ventilation (NIV) tube 40. An airtight interface or passageway for gas flow is provided between second end portion 120 of connector 100 and NIV tube 40. A second end of NIV tube 40 is connectable to a mechanical ventilator (not shown). An elbow portion 130 is disposed between first end portion 110 and second end portion 120 of connector 100. NIV therapy may be provided to the patient by the mechanical ventilator (not shown) and respiratory mask 10, via NIV tube 40; second end portion 120, elbow portion 130 and first end portion 110 of connector 100; and hub 30.

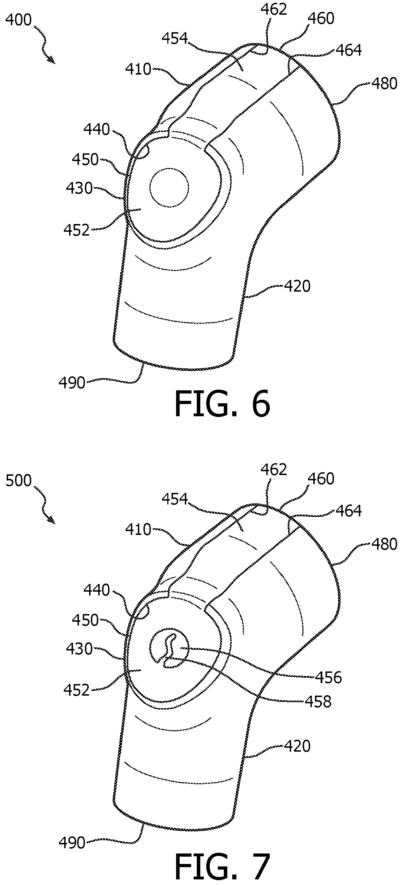

Elbow portion 130 includes port 140 having a removable seal 150 fit into port 140. In a representative embodiment, removable seal 150 may include a slit (such as shown in FIG. 7) through which a first end of tube 50 is insertable into connector 100. Tube 50 may be an endotracheal tube for supplying gas, and may include a second end connectable with the mechanical ventilator. Tube 50 may be inserted into the mouth of the patient via removable seal 150 of port 140, connector 100, hub 30 and respiratory mask 10, to provide invasive ventilation (IV) therapy. In a representative embodiment, connector 100 may be plastic, and may be formed by injection molding and ultrasonic welding for example. Removable seal 150 may be a pliable or flexible sealing material such as silicone and/or thermoplastic polyurethane. In the case that removable seal 150 includes a slit, removable seal 150 may be pliable or flexible to conform around the exterior circumference of tube 50, providing a substantially airtight seal so as to maintain NIV therapy via NIV tube 40 during insertion of tube 50. As will be subsequently described with reference to FIG. 2, first end portion 110 may be hingeable so as to be split apart and openable along joint 160 which extends from the first opening of first end portion 110 to port 140 at elbow portion 130, to enable removal of connector 100 from around tube 50 inserted into port 140.

FIG. 2 is a diagram illustrating a connector with a hingeable end portion, according to a representative embodiment.

Referring to FIG. 2, connector 100 is shown as including similar features as shown in FIG. 1 respectively denoted by similar reference numerals. Connector 100 in FIG. 2 is in a state removed from hub 30 of respiratory mask 10, with second end portion 120 connected to NIV tube 40. First end portion 110 is split or opened along joint 160 (shown in FIG. 1) and is in a state removed from around tube 50 shown in FIG. 1 (which may hereinafter be referred to as the inserted tube). As opened, first end portion 110 of connector 100 includes integral hinge 170 providing pivotable connection between first section 112 and second section 114 of first end portion 110. Mating surface 162 of first section 112 and mating surface 164 of second section 114 abut and are held against each other at joint 160 in a closed state as shown in FIG. 1. Joint 160 as formed by mating surfaces 162 and 164 extends from first opening 180 of first end portion 110 shown in FIG. 2, to port 140 at elbow portion 130. When opened at joint 160, connector 100 is removable from around tube 50 previously inserted into port 140. To further understanding, removable seal 150 is not shown in FIG. 2.

Transition from non-invasive ventilation to invasive ventilation in accordance with a representative embodiment is hereafter described with reference to FIGS. 1 and 2. During NIV therapy, the second end (not shown) of NIV tube 40 may be connected to a mechanical ventilator to provide appropriate pressure and volume of oxygen, gases and/or gas mixtures. In the event that patient respiratory stress continues or worsens, or in the case of preparation for major surgery, tube 50 (which may be an endotracheal tube) may be inserted into port 140 through removable seal 150, subsequently manipulated through connector 100 and hub 30, and then inserted directly into the upper lungs through the mouth of the patient. Thereafter, connector 100 may be removed from hub 30, and first end portion 110 may be opened along joint 160 by way of hinge 170, splitting first end portion 110 into first section 112 and second section 114. In an opened state, connector 100 may then be removed from around tube 50. Respiratory mask 10 may then be removed from the patient, and slid along and off the unconnected second end of tube 50. Thereafter in a final step, the second end of tube 50 may be connected directly to the mechanical ventilator. In an alternative final step, while the second end (not shown) of NIV tube 40 is maintained as connected to the mechanical ventilator, the first end of NIV tube 40 may be disconnected from second end portion 120 of connector 100, and tube 50 may be connected to the first end of NIV tube 40 via an adaptor. In accordance with the representative embodiments, an efficient transition from non-invasive ventilation to invasive ventilation may be realized without substantial interruption of ventilation.

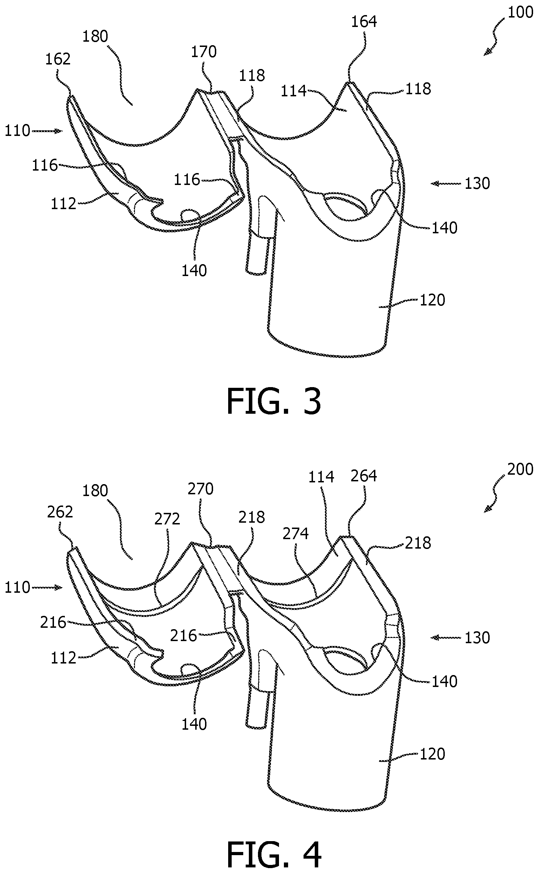

FIG. 3 is a diagram illustrating a connector with a hingeable end portion having mating surfaces respectively including ridges and recesses, according to a representative embodiment.

Referring to FIG. 3, connector 100 is shown as including similar features as in FIG. 2 respectively denoted by similar reference numerals, and redundant description of such similar features may be omitted for brevity. Connector 100 in FIG. 3 is in a state removed from hub 30 of respiratory mask 10. To further understanding, removable seal 150 and NIV tube 40 are not shown in FIG. 3. As opened, first end portion 110 of connector 100 includes integral hinge 170 providing pivotable connection between first section 112 and second section 114 of first end portion 110. Connector 100 is in a state removed from around an inserted tube such as tube 50 shown in FIG. 1. First section 112 of first end portion 110 has respective integral ridges 116 protruding from mating surfaces 162 on both lateral sides of first section 112 including near hinge 170. Second section 114 of first end portion 110 has respective recesses 118 formed in mating surfaces 164 on both lateral sides of second section 114 including near hinge 170. In a representative embodiment, connector 100 may be made of molded plastic, with ridges 116 and recesses 118 formed as having interference geometries with extension and dimension so that ridges 116 fit into recesses 118 with temporary retaining force that holds first section 112 and second section 114 together when first end portion 110 is in a closed state. Ridges 116 and recesses 118 together define openable snap-fit joints between first section 112 and second section 114 of first end portion 110. In further representative embodiments, different interference geometries such as interlocking block teeth, dovetails or keystone may be used instead of continuous ridges and recesses. Also, other types of snap-fit joints such as cantilever snap-fit joints and hook/recess snap-fit joints may be used. As a further alternative, a mild adhesive may be applied to mating surface 162 of first section 112 and to mating surface 164 of second section 114, to hold the first and second sections 112 and 114 together when first end portion 110 is in a closed state.

FIG. 4 is a diagram illustrating a connector with a hingeable end portion having mating surfaces respectively including ridges and recesses made of flexible sealing material, according to a representative embodiment.

Referring to FIG. 4, connector 200 is shown as including similar features as in FIG. 3 respectively denoted by similar reference numerals, and redundant description of such similar features may be omitted for brevity. Connector 200 in FIG. 4 is in a state removed from hub 30 of respiratory mask 10. To further understanding, removable seal 150 and NIV tube 40 are not shown in FIG. 4. Connector 200 includes mating surfaces 262 of first section 112 and mating surfaces 264 of second section 114, and hinge 270 made of flexible sealing material such as silicone and/or thermoplastic polyurethane. As opened, first end portion 110 of connector 200 includes integral hinge 270 providing pivotable connection between first section 112 and second section 114 of first end portion 110. Connector 200 is in a state removed from around an inserted tube such as tube 50 shown in FIG. 1. First section 112 of first end portion 110 has respective integral ridges 216 protruding from mating surfaces 262 on both lateral sides of first section 112 including near hinge 270. Second section 114 of first end portion 110 has respective recesses 218 formed in mating surfaces 264 on both lateral sides of second section 114 including near hinge 270. In a representative embodiment, connector 200 may be made of molded plastic, and mating surfaces 262 and 264 respectively including ridges 216 and recesses 218, and hinge 270 pivotably connecting first section 112 and second section 114, may be formed of silicone overmolded on first section 112 and second section 114. Incidentally, band 272 on the inner surface of first section 112 and band 274 on the inner surface of second section 114 are formed during the overmolding process. Ridges 216 and recesses 218 may be formed during the overmolding process as having interference geometries with extension and dimension so that ridges 216 fit into recesses 218 with temporary retaining force that holds first section 112 and second section 114 together when first end portion 110 is in a closed state. Ridges 216 and recesses 218 together define openable snap-fit joints between first section 112 and second section 114 of first end portion 110. As described previously, in further representative embodiments, interference geometries such as interlocking teeth, dovetails and keystone may be used instead of continuous ridges and recesses, and also mild adhesive may be used in still further representative embodiments.

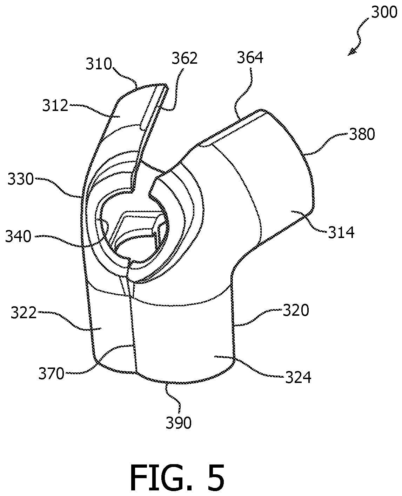

FIG. 5 is a diagram illustrating a connector that is hingeable and having first and second end portions that are openable, according to a representative embodiment.

Referring to FIG. 5, connector 300 is shown as including similar features as shown in FIG. 2 respectively denoted by similar reference numerals. Connector 300 in FIG. 5 is in a state removed from hub 30 of respiratory mask 10 and removed from NIV tube 40 (shown in FIG. 1). To further understanding, removable seal 150 is not shown in FIG. 5. First end portion 310 is split or opened, and is in a state removed from around an inserted tube such as tube 50 shown in FIG. 1. As opened, first end portion 310 of connector 300 is split from first opening 380 to port 340 at elbow portion 330 along both the top and bottom, and includes first section 312 and second section 314. Mating surface 362 of first section 312 and mating surface 364 of second section 314 at the top of first end portion 310 abut and are held against each other in a closed state. Similarly, mating surfaces (not shown) of first section 312 and second section 314 along the bottom of first end portion 310 abut and are held against each other in the closed state. The corresponding mating surfaces of first and second sections 312 and 314 may include interference geometries providing snap-fit joints as described with respect to FIG. 3. Second end portion 320 includes hinge 370 along a front of connector 300, extending from opening 390 to port 340. As opened, second end portion 320 is split with hinge 370 providing connection between first section 322 and second section 324. Mating surfaces (not shown) of first section 322 and second section 324 along the back of second end portion 320 abut and are held against each other in a closed state, and may include corresponding interference geometries. Mating surfaces (not shown) of first section 322 and second section 324 along the front of second end portion 320 may also include corresponding interference geometries.

FIG. 6 is a diagram illustrating a connector having a channel and a port, including a removable seal in the channel and port, according to a representative embodiment.

Referring to FIG. 6, connector 400 is in a state prior to insertion of a tube such as tube 50 shown in FIG. 1. To further understanding, hub 30, respiratory mask 10 and NIV tube 40 are not shown in FIG. 6. Connector 400 includes first end portion 410 having first opening 480 removably insertable into hub 30 of respiratory mask 10. Second end portion 420 of connector 400 includes second opening 490 and is removably connectable to NIV tube 40. Elbow portion 430 is disposed between first end portion 410 and second end portion 420. Elbow portion 430 includes port 440 through which a first end of a tube such as tube 50 shown in FIG. 1 may be inserted. First end portion 410 further includes a channel 460 extending along the top from first opening 480 to port 440. First end portion 410 may be of general tubular cross-section, discontinuous at channel 460. Channel 460 may be defined by opposing first and second edge surfaces 462 and 464 of first end portion 410. Removable seal 450 may be an integral seal including first seal portion 452 fit within port 440 and second seal portion 454 fit within channel 460. Removable seal 450 may be a flexible, pliable material such as silicone and/or thermoplastic polyurethane.

In a representative embodiment, during non-invasive ventilation while connector 400 is connected to NIV tube 40 and respiratory mask 10 via hub 30, removable seal 450 including first and second seal portions 452 and 454 may be removed from port 440 and channel 460, and a tube such as tube 50 shown in FIG. 1 may then be inserted into connector 400 via port 440 and channel 460. Prior to further manipulation of the inserted tube, second seal portion 454 may be urged back into channel 460, and first seal portion 452 may then be closed down and around the tube inserted within port 440, to maintain adequate gas flow to the patient as non-invasive ventilation continues. The inserted tube may then be further manipulated to be fully inserted into the patient through connector 400 and respiratory mask 10. Thereafter, removable seal 450 including first and second seal portions 452 and 454 may be removed from port 440 and channel 460, and connector 400 may be removed from around the fully inserted tube and from hub 30 of respiratory mask 10. Respiratory mask 10 may then be removed from the patient, and slid off along the inserted tube. A second end of the inserted tube may then be connected to the mechanical ventilator as described previously with respect to FIG. 2, to begin invasive ventilation. Removable seal 450 including first and second seal portions 452 and 454 may then be urged back into port 440 and channel 460 of removed connector 400.

FIG. 7 is a diagram illustrating a connector having a channel and a port, including a removable seal in the channel and port, the removable seal in the port having a slit, according to a representative embodiment.

Referring to FIG. 7, connector 500 is shown as including similar features as in FIG. 6 respectively denoted by similar reference numerals, and description of such similar features may be omitted for brevity. Removable seal 450 may be made of a flexible, pliable material, and may be an integral seal including first seal portion 452 fit within port 440 and second seal portion 454 fit within channel 460. First seal portion 452 includes a central section 456 having slit 458 extending from a bottom of central section 456 vertically upward as shown. The bottom portion of central section 456 may be detachable from first seal portion 452, while the top portion of central section 456 may be integrally formed as connected to first seal portion 452. Central section 456 of removable seal 450 may be characterized as an openable flap.

In a representative embodiment, during non-invasive ventilation while connector 500 is connected to NIV tube 40 and respiratory mask 10 via hub 30, the bottom portion of central section 456 may be detached from first seal portion 452, and a tube such as tube 50 shown in FIG. 1 may then be inserted into connector 400 via port 440. Prior to further manipulation of the inserted tube, the bottom portion of central section 456 may be urged back down around the inserted tube and into connection with first seal portion 452. Since removable seal 450 is made of a flexible, pliable material, central section 456 may be urged at slit 458 to conform around the exterior circumference of the inserted tube, to maintain adequate gas flow to the patient as non-invasive ventilation continues. The inserted tube may then be further manipulated to be inserted into the patient through connector 500 and respiratory mask 10. Thereafter, removable seal 450 including first and second seal portions 452 and 454 may be removed from port 440 and channel 460, and connector 500 may be removed from around the fully inserted tube and from hub 30 of respiratory mask 10. Respiratory mask 10 may then be removed from the patient, and slid off along the inserted tube along with removable seal 450. A second end of the inserted tube may then be connected to the mechanical ventilator as described previously with respect to FIG. 2, to begin invasive ventilation. Removable seal 450 including first and second seal portions 452 and 454 may then be urged back into port 440 and channel 460 of removed connector 500.

In the representative embodiments, connector 100 is described with reference to FIG. 2 for example as enabling efficient transition from non-invasive ventilation to invasive ventilation without substantial interruption of ventilation. As a further application, the connectors of the representative embodiments may be used to superoxygenate and provide inhaled anesthetic agents to the patient for induction and at the same time open the patient airway, using non-invasive ventilation. Intubation may then follow. That is, the patient airway may be opened for intubation using non-invasive ventilation without a laryngoscope.

In the representative embodiments of FIGS. 6 and 7, removable seal 450 may be secured to the edge of the connector at port 440 by any of a variety of mechanical interlock mechanisms as would be understood by one of ordinary skill. For example, a ribbed section may protrude from the underside of removable seal 450 which may engage and interlock with a ridge section formed at the edge of the connector at port 440. As a further example, removable seal 450 and the edge of the connector at port 440 may be respectively made of silicone and plastic having chemical properties so that removable seal 450 adheres to the edge of the connector at port 440.

Also, the connectors of the representative embodiments have been described as including an elbow portion having a port, disposed between opposite first and second end portions having an angle of about 90° with respect to each other. Connectors of other representative embodiments may include junction portions disposed between opposite first and second end portions having any one of a variety of angles other than 90° with respect to each other.

While several representative embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. For example, the connectors of the representative embodiments may be used to enable insertion of tubes other than endotracheal tubes into the patient, and also may be used in connection with carbon dioxide sensors, oxygen sensors and/or pressure sensors.

Those skilled in the art should also readily appreciate that all parameters, dimensions, materials, and configurations described herein are meant to be exemplary and that the actual parameters, dimensions, materials, and/or configurations will depend upon the specific application or applications for which the representative embodiments is/are used. Those skilled in the art will recognize, or be able to ascertain using no more than routine experimentation, many equivalents to the specific representative embodiments described herein. It is, therefore, to be understood that the foregoing embodiments are presented by way of example only and that, within the scope of the appended claims and equivalents thereto, representative embodiments may be practiced otherwise than as specifically described and claimed. Representative embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

## Claims

1. A connector (110) for a respiratory mask (10), the connector comprising:
a first end portion (110) defining a first opening to connect with the respiratory mask;
a second end portion (120) defining a second opening to receive a non-invasive (NIV) tube (40); and
a junction portion (130) between the first and second end portions, the junction portion defining a port (140) for receiving an endotracheal tube (50),
wherein at least the first end portion and the junction portion are openable for removal of the connector from around the endotracheal tube inserted in the port.

2. The connector of claim 1, wherein the first end portion (110) includes a hinge (170) and is configured to be openable.

3. The connector of claim 2, wherein the first end portion (110) comprises first and second sections (112, 114) connected together by the hinge (170), mating surfaces (162, 164) of the first and second sections configured to respectively include ridges (116) and recesses (118) defining an openable snap-fit joint (160) between the first and second sections.

4. The connector of claim 3, wherein the mating surfaces of the first and second sections (112, 114) are comprised of a flexible sealing material.

5. The connector of claim 4, wherein the flexible sealing material is silicone.

6. The connector of claim 2, wherein the first end portion (110), the second end portion (120), the junction_portion (130) and the hinge (170) are integrally molded.

7. The connector of claim 1, further comprising a removable seal (150) that is configured to fit in the port (140).

8. The connector of claim 7, wherein the first end portion (110) includes a channel (460) extending from the first opening (480) to the port (440), the removable seal (150) further configured to fit in the channel.

9. The connector of claim 7, wherein the removable seal (150) includes a slit (458) for receiving the tube.

10. The connector of claim 7, wherein the removable seal (150) is comprised of silicone.

11. The connector of claim 1, wherein the second end portion (120) includes a hinge (170), and both the first end portion (110) and the second end portion (120) are configured to be openable.

12. The connector of claim 1, wherein the first end portion (110), the second end portion (120) and the junction portion (130) are integrally molded.

13. The connector of claim 1, wherein the junction portion (130) is configured as an elbow portion.

14. The connector of claim 14, wherein the first end portion includes a channel (460) extending from the first opening to the port (140).

## Patentansprüche

1. Konnektor (110) für eine Atemmaske (10), wobei der Konnektor Folgendes umfasst:
einen ersten Endabschnitt (110), der eine erste Öffnung für die Verbindung mit der Atemmaske definiert;
einen zweiten Endabschnitt (120), der eine zweite Öffnung zum Empfangen eines nicht-invasiven (NIV) Tubus (40) definiert; und
einen Übergangsabschnitt (130) zwischen dem ersten und dem zweiten Endabschnitt, wobei der Übergangsabschnitt einen Anschluss (140) zum Empfangen eines Endotrachealtubus (50) definiert,
wobei mindestens der erste Endabschnitt und der Übergangsabschnitt geöffnet werden können, um den Konnektor um den in den Anschluss eingeführten Endotrachealtubus herum entfernen zu können.

2. Konnektor nach Anspruch 1, wobei der erste Endabschnitt (110) ein Gelenk (170) umfasst und konfiguriert ist, um geöffnet werden zu können.

3. Konnektor nach Anspruch 2, wobei der erste Endabschnitt (110) erste und zweite Sektionen (112, 114) umfasst, die durch das Gelenk (170) miteinander verbunden sind, wobei Fügeflächen (162, 164) der ersten und der zweiten Sektion konfiguriert sind, um jeweils Grate (116) und Vertiefungen (118) zu umfassen, die eine zu öffnende Schnappverbindung (160) zwischen der ersten und der zweiten Sektion definieren.

4. Konnektor nach Anspruch 3, wobei die Fügeflächen der ersten und der zweiten Sektion (112, 114) aus einem flexiblen Dichtungsmaterial bestehen.

5. Konnektor nach Anspruch 4, wobei das flexible Dichtungsmaterial Silikon ist.

6. Konnektor nach Anspruch 2, wobei der erste Endabschnitt (110), der zweite Endabschnitt (120), der Übergangsabschnitt (130) und das Gelenk (170) integral geformt sind.

7. Konnektor nach Anspruch 1, weiterhin umfassend eine entfernbare Dichtung (150), die konfiguriert ist, um in den Anschluss (140) zu passen.

8. Konnektor nach Anspruch 7, wobei der erste Endabschnitt (110) einen Kanal (460) umfasst, der sich von der ersten Öffnung (480) zu dem Anschluss (440) erstreckt, wobei die entfernbare Dichtung (150) weiterhin konfiguriert ist, um in den Kanal zu passen.

9. Konnektor nach Anspruch 7, wobei die entfernbare Dichtung (150) einen Schlitz (458) zum Aufnehmen des Tubus umfasst.

10. Konnektor nach Anspruch 7, wobei die entfernbare Dichtung (150) aus Silikon besteht.

11. Konnektor nach Anspruch 1, wobei der zweite Endabschnitt (120) ein Gelenk (170) umfasst, und sowohl der erste Endabschnitt (110) als auch der zweite Endabschnitt (120) konfiguriert sind, um geöffnet werden zu können.

12. Konnektor nach Anspruch 1, wobei der erste Endabschnitt (110), der zweite Endabschnitt (120) und der Übergangsabschnitt (130) integral geformt sind.

13. Konnektor nach Anspruch 1, wobei der Übergangsabschnitt (130) als ein Knieabschnitt konfiguriert ist.

14. Konnektor nach Anspruch 14, wobei der erste Endabschnitt einen Kanal (460) umfasst, der sich von der ersten Öffnung zum Anschluss (140) erstreckt.

## Revendications

1. Raccord (110) pour un masque respiratoire (10), le raccord comprenant :
une première partie terminale (110) définissant une première ouverture pour un raccordement avec le masque respiratoire ;
une deuxième partie terminale (120) définissant une deuxième ouverture pour recevoir un tube (40) non invasif (NIV); et
une partie de jonction (130) entre les première et deuxième parties terminales, la partie de jonction définissant un orifice (140) pour recevoir un tube endotrachéal (50),
dans lequel au moins la première partie terminale et la partie de jonction peuvent être ouvertes pour retirer le raccord situé autour du tube endotrachéal inséré dans l'orifice.

2. Raccord selon la revendication 1, dans lequel la première partie terminale (110) comprend une charnière (170) et est configurée pour pouvoir être ouverte.

3. Raccord selon la revendication 2, dans lequel la première partie terminale (110) comprend des première et deuxième sections (112, 114) raccordées l'une à l'autre par la charnière (170), des surfaces d'accouplement (162, 164) des première et deuxième sections configurées de façon à comprendre respectivement des arêtes (116) et des cavités (118) définissant un joint à encliquetage pouvant être ouvert (160) entre les première et deuxième sections.

4. Raccord selon la revendication 3, dans lequel les surfaces d'accouplement des première et deuxième sections (112, 114) sont constituées d'un matériau de scellement souple.

5. Raccord selon la revendication 4, dans lequel le matériau de scellement souple est la silicone.

6. Raccord selon la revendication 2, dans lequel la première partie terminale (110), la deuxième partie terminale (120), la partie de jonction (130) et la charnière (170) sont moulées en une seule pièce.

7. Raccord selon la revendication 1, comprenant en outre un joint d'étanchéité amovible (150) qui est configuré pour s'ajuster dans l'orifice (140).

8. Raccord selon la revendication 7, dans lequel la première partie terminale (110) comprend un canal (460) s'étendant de la première ouverture (480) à l'orifice (440), le joint d'étanchéité amovible (150) étant en outre configuré pour s'ajuster dans le canal.

9. Raccord selon la revendication 7, dans lequel le joint d'étanchéité amovible (150) comprend une fente (458) pour recevoir le tube.

10. Raccord selon la revendication 7, dans lequel le joint d'étanchéité amovible (150) est constitué de silicone.

11. Raccord selon la revendication 1, dans lequel la deuxième partie terminale (120) comprend une charnière (170) et la première partie terminale (110) et la deuxième partie terminale (120) sont toutes deux configurées de façon à pouvoir être ouvertes.

12. Raccord selon la revendication 1, dans lequel la première partie terminale (110), la deuxième partie terminale (120) et la partie de jonction (130) sont moulées en une seule pièce.

13. Raccord selon la revendication 1, dans lequel la partie de jonction (130) est configurée comme une partie coudée.

14. Raccord selon la revendication 14, dans lequel la première partie terminale comprend un canal (460) s'étendant de la première ouverture à l'orifice (140).
